# EUROPEAN PATENT SPECIFICATION

(11) **EP 1 270 500 B1**
(45) Date of publication and mention of the grant of the patent: **08.12.2004**
(21) Application number: 02014335.0
(22) Date of filing: 27.06.2002
(51) Int. Cl.: B67D 1/00, B67D 3/00, A61L 2/10

(54) **Unit for dispensing liquid food products**
Vorrichtung zur Ausgabe von flüssigen Lebensmitteln
Unité de distribution de produits alimentaires liquides

(30) Priority: 29.06.2001 IT BO20010413
(43) Date of publication of application: 02.01.2003
(73) Proprietor: Cosmetal Sistemi Di Refrigerazione S.r.l., 62019 Recanati (IT)
(72) Inventor: Grottini, Gianni, 60025 Loreto (IT)
(74) Representative: Cerbaro, Elena

(56) References cited:
- WO-A-00/59821
- US-A- 4 276 256
- US-A- 4 322 291
- US-A- 4 342 915
- US-A- 5 053 143
- US-A- 5 316 673

## Description

The present invention relates to a unit for dispensing liquid food products.

More specifically, the present invention relates to a unit for dispensing drinking water, to which the following description refers purely by way of example.

For dispensing drinking water, a dispensing unit is used comprising at least one outlet, and a hydraulic circuit connecting the outlet to a water source.

Document US 5,316,673 discloses a dispensing unit comprising one outlet, a hydraulic circuit connecting the outlet to a water source, and an ultraviolet lamp mounted parallel to and adjacent a section of the hydraulic circuit.

Document WO 00/59821 discloses a machine for dispensing a foodstuff comprising one outlet, a hydraulic circuit connecting the outlet to a foodstuff source, and sterilizing means for sterilizing a space enclosing the outlet and the outlet itself according to the preambles of claims 1 and 4.

A major drawback of known units of the above type lies in the outlet, since it must be located in a position accessible to the user, being easily contaminated by microorganisms such as bacteria, mold, ferments and/or protozoa present in the air and/or on the user's hands, and which may therefore contaminate the water dispensed.

It is an object of the present invention to provide a unit for dispensing liquid food products, designed to eliminate the aforementioned drawbacks.

According to the present invention, there is provided a unit for dispensing at least one liquid food product as recited in Claim 1.

A non-limiting embodiment of the present invention will be described by way of example with reference to the accompanying drawings, in which:
Figure 1 shows a schematic view in perspective of a preferred embodiment of the unit for dispensing liquid food products according to the present invention;
Figure 2 shows a front view, with parts removed for clarity, of the Figure 1 unit;
Figure 3 shows a side view, with parts removed for clarity, of the Figure 1 unit;
Figure 4 shows an exploded view in perspective of a dispensing kit that can be fitted to an existing unit for dispensing liquid food products;
Figure 5 shows a schematic side view of the Figure 4 kit fitted to an existing unit for dispensing liquid food products.

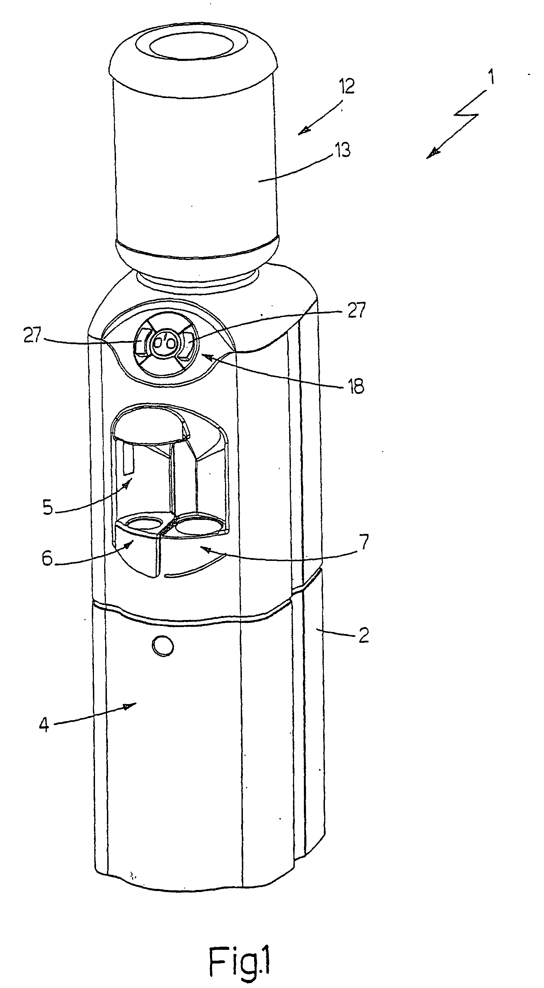

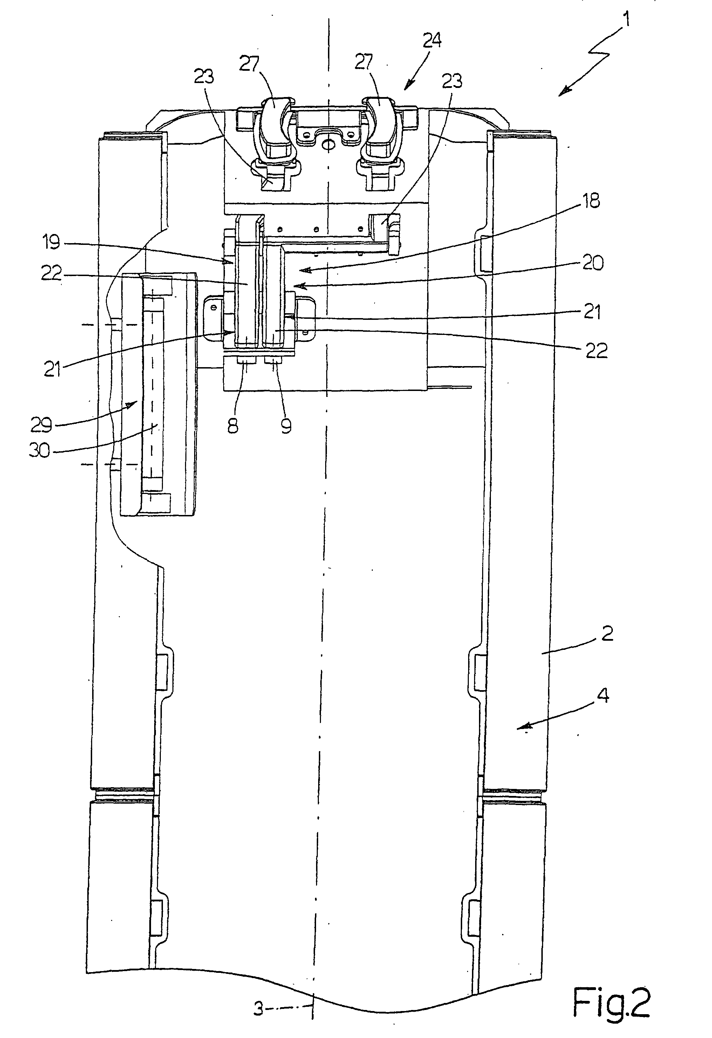

Number 1 in Figure 1 indicates as a whole a unit for dispensing liquid food products - in the example shown, drinking water - and comprising a substantially tubular outer casing 2, which has a substantially vertical longitudinal axis 3 (Figure 2), is defined at the front by a front surface 4, and has a cavity 5 opening outwards at surface 4.

Cavity 5 is provided at the bottom with a water collecting tank 6 and a cup dispenser 7, and at the top with at least one water outlet - in the example shown, two outlets 8, 9 (Figure 2) extending downwards parallel to axis 3, and for dispensing cold and hot water respectively.

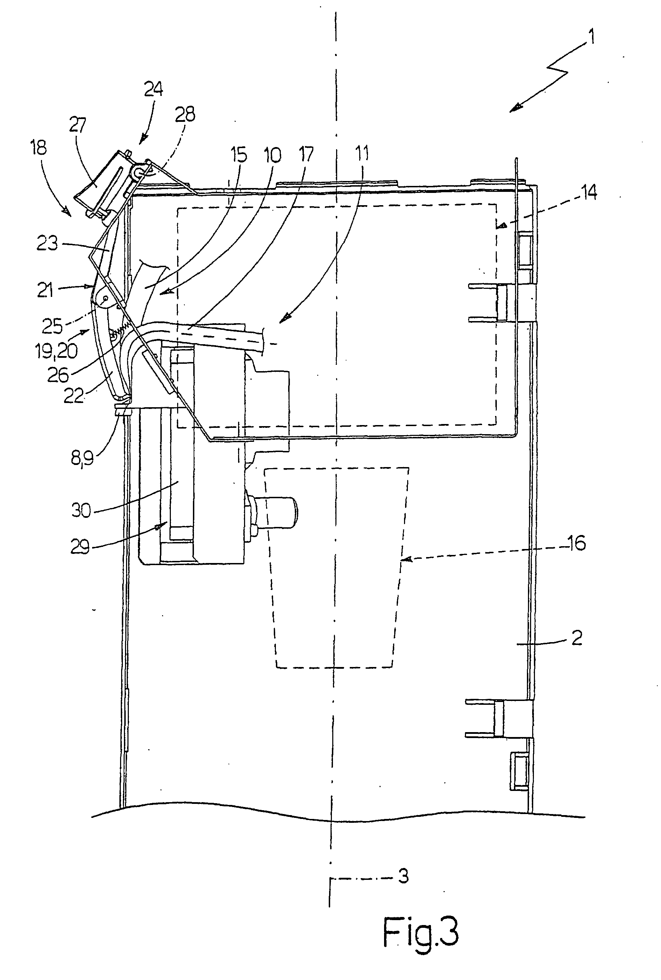

Outlets 8, 9 are connected by respective hydraulic circuits 10, 11 (Figure 3) to a drinking water source 12 defined, in the example shown, by a substantially cup-shaped tank 13 fitted removably to the top portion of casing 2 in an upside down position so that the water flows down into circuits 10, 11. In a variation not shown, tank 13 may be eliminated, and circuits 10, 11 may be connected directly to the water mains which thus defines source 12.

With reference to Figure 3, circuit 10 comprises a tank 14 for storing cold water and having a known cooling device (not shown); a conduit (not shown) hydraulically connecting tanks 13 and 14; and a conduit 15 hydraulically connecting tank 14 and outlet 8. Circuit 11 comprises a tank 16 for storing hot water and having a known heating device (not shown); a conduit (not shown) hydraulically connecting tanks 13 and 16; and a conduit 17 hydraulically connecting tank 16 and outlet 9.

With reference to Figures 2 and 3, water supply from tanks 14, 16 to respective outlets 8, 9 is controlled selectively by a control device 18 comprising two shutters 19, 20 associated with outlets 8, 9 respectively, and each of which comprises a respective rocker arm 21 having a bottom arm 22 and a top arm 23, and hinged to casing 2 to rotate, with respect to casing 2 and by virtue of a relative actuating device 24, about a hinge axis 25 substantially crosswise to axis 3 and also shared by the other rocker arm 21.

Device 24 comprises a spring 26 interposed between arm 22 and casing 2 to keep rocker arm 21 normally in a closed position (Figure 3) in which a free end of arm 22 engages a free end of relative conduit 15, 17 to cut off water flow by choking relative conduit 15, 17.

Device 24 also comprises a control button 27, which is located over rocker arm 21, is hinged to casing 2 to rotate, with respect to casing 2, about a hinge axis 28 substantially parallel to axis 25 and also shared by the other button 27, and engages a free end of arm 23 to move rocker arm 21, in opposition to spring 26, into an open position (not shown) in which the free end of arm 22 is located a given distance from relative conduit 15, 17 to permit water supply to relative outlet 8, 9.

With reference to Figures 1, 2 and 3, unit 1 also comprises a sterilizing device 29 in turn comprising an ultraviolet lamp 30, which is housed in casing 2, extends substantially parallel to axis 3, and is kept on permanently to irradiate and sterilize outlets 8, 9 and a space enclosing outlets 8, 9. More specifically, lamp 30 emits ultraviolet radiation of a wavelength substantially below 320 nanometers, and preferably ranging between 240 and 320 nanometers, to destroy microorganisms such as bacteria, mold, ferments and/or protozoa on outlets 8, 9 and in the air surrounding outlets 8, 9.

In a variation not shown, device 29 may comprise a sensor for detecting the presence of a user about to draw water from unit 1; and a switch for turning lamp 30 on and/or off as a function of a signal from the sensor.

Operation of unit 1 is easily deducible from the foregoing description, with no further explanation required.

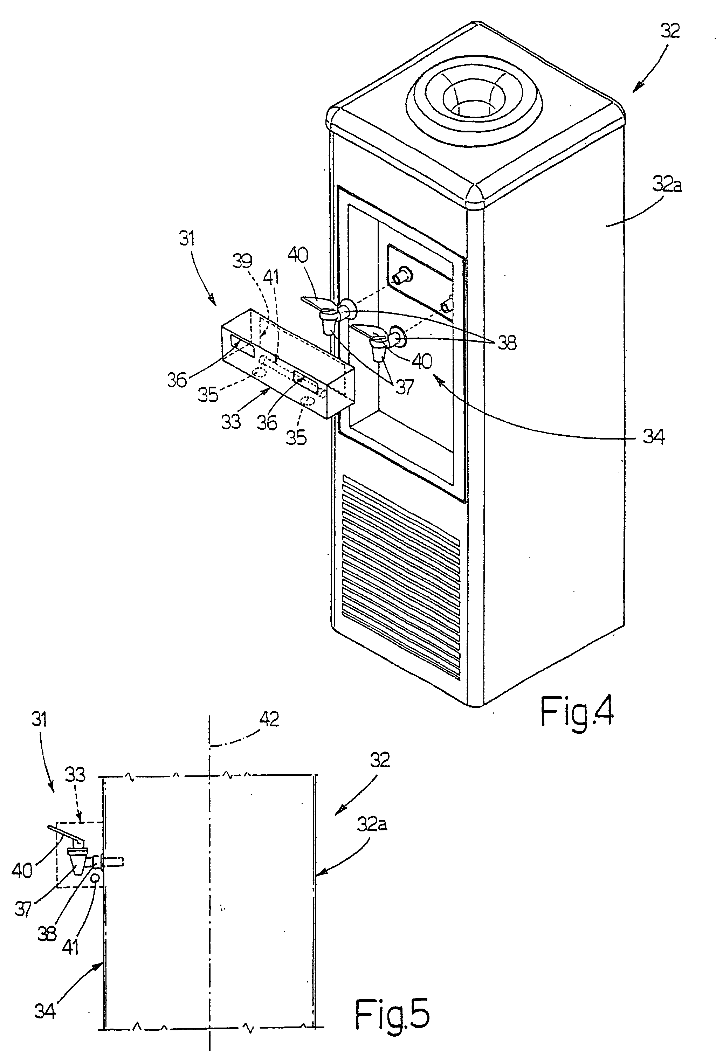

Figures 4 and 5 show a kit 31 which can be fitted to an existing unit 32 for dispensing liquid food products, and comprises a box-shaped body 33, which is substantially in the form of a substantially rectangular-section parallelepiped, can be fitted to a front surface 34 of a casing 32a of unit 32 by means of fastening members not shown, and has two bottom outlet holes 35 and two front openings 36, all formed through body 33 itself.

Kit 31 also comprises two outlets 37, which are housed inside body 33, substantially coaxially with holes 35, have respective fittings 38 extending through a rear opening 39 in body 33 to connect outlets 37 to respective hydraulic circuits (not shown) of unit 32, and have respective operating levers 40 projecting outwards of body 33 through openings 36.

Kit 31 also comprises an ultraviolet lamp 41 identical with lamp 30 and housed inside body 33, crosswise to a longitudinal axis 42 of unit 32, to irradiate and sterilize the two outlets 37 and a space enclosing outlets 37.

## Claims

1. A unit for dispensing at least one liquid food product, the unit comprising at least one outlet (8, 9), a hydraulic circuit (10, 11) being suitable for connecting said outlet (8, 9) to a source (12) of said product, and sterilizing means (29) for sterilizing a space enclosing said outlet (8, 9) and the outlet (8, 9) itself; and being **characterized in that** said sterilizing means (29) comprise at least one ultraviolet lamp (30) for irradiating said outlet (8, 9) and said space enclosing said outlet.

2. A unit as claimed in Claim 1, wherein the rays of said ultraviolet lamp have a wavelength substantially below 320 nanometers.

3. A unit as claimed in Claim 1 or 2, and also comprising control means for detecting the presence, at said outlet (8, 9), of a user of said unit; actuating means being provided to turn said ultraviolet lamp (30) on and/or off as a function of a signal from said control means.

4. A kit for dispensing at least one liquid food product, the kit comprising at least one outlet (37); sterilizing means (41) for sterilizing a space enclosing said outlet (37) and the outlet (37) itself; and supporting means (33) for supporting at least said sterilizing means (41); said outlet (37) and said supporting means (33) being connectable respectively to a hydraulic circuit and a casing (32a) of a unit (32) for dispensing liquid food products; and being **characterized in that** said sterilizing means (41) comprise at least one ultraviolet lamp (41) for irradiating said outlet (37) and said space enclosing said outlet.

## Patentansprüche

1. Baueinheit zum Ausgeben vom wenigstens einem flüssigen Nahrungsmittelprodukt, welche Baueinheit wenigstens einen Auslass (8, 9), einen Hydraulikkreislauf (10, 11) der geeignet ist, den Auslass (8, 9) mit einer Quelle (12) des besagten Produktes zu verbinden, und Sterilisierungseinrichtungen (29) zum Sterilisieren eines Raumes, der den Auslass (8, 9) umschließt und des Auslasses (8, 9) selbst umfasst, **dadurch gekennzeichnet, dass** die Sterilisierungseinrichtungen (29) wenigstens eine ultraviolette Lampe (30) zum Bestrahlen des Auslasses (8, 9) und des Raumes, der den Auslass umschließt, umfasst.

2. Baueinheit nach Anspruch 1, bei der die Strahlung der ultravioletten Lampe eine Wellenlänge hat, die im Wesentlichen unter 320 Nanometern liegt.

3. Baueinheit nach Anspruch 1 oder 2, welche gleichfalls Steuereinrichtungen umfasst, um das Vorhandensein eines Benutzers der Baueinheit am Auslass (8, 9) festzustellen, wobei Betätigungseinrichtungen vorgesehen sind, um die ultraviolette Lampe (30) in Abhängigkeit vom einem Signal von den Steuereinrichtungen an- und/oder auszuschalten.

4. Bausatz zum Ausgeben wenigstens eines flüssigen Nahrungsmittelproduktes, welcher Bausatz wenigstens einen Auslass (37), Sterilisierungseinrichtungen (41) zum Sterilisieren eines Raumes, der den Auslass (37) umschließt, und des Auslasses (37) selbst und Halteeinrichtungen (33) zum Halten wenigstens der Sterilisierungseinrichtungen (41) umfasst, wobei der Auslass (37) und die Halteeinrichtungen (33) jeweils mit einem Hydraulikkreislauf und einem Gehäuse (32a) einer Baueinheit (32) zum Ausgeben flüssiger Nahrungsmittelprodukte verbindbar sind, **dadurch gekennzeichnet, dass** die Sterilisierungseinrichtungen (41) wenigstens eine ultraviolette Lampe (41) umfassen, um den Auslass (37) und den Raum, der den besagten Auslass umgibt, zu bestrahlen.

## Revendications

1. Unité de distribution d'au moins un produit alimentaire liquide, l'unité comprenant au moins une sortie (8, 9), un circuit hydraulique (10, 11) étant agencé pour relier ladite sortie (8, 9) à une source (12) dudit produit, et des moyens de stérilisation (29) pour stériliser un espace entourant ladite sortie (8, 9) et la sortie (8, 9) elle-même; et étant **caractérisée en ce que** lesdits moyens de stérilisation (29) comprennent au moins une lampe ultraviolette (30) pour irradier ladite sortie (8, 9) et ledit espace entourant ladite sortie.

2. Unité telle que revendiquée dans la revendication 1, dans laquelle les rayons de ladite lampe ultraviolette ont une longueur d'onde sensiblement inférieure à 320 nanomètres.

3. Unité telle que revendiquée dans la revendication 1 ou 2, et comprenant également des moyens de contrôle pour détecter la présence, à ladite sortie (8, 9), d'un utilisateur de ladite unité, des moyens d'activation étant ménagés pour allumer et/ou éteindre ladite lampe ultraviolette (30) en fonction d'un signal desdits moyens de contrôle.

4. Equipement pour distribuer au moins un produit alimentaire liquide, l'équipement comprenant au moins une sortie (37); des moyens de stérilisation (41) pour stériliser un espace entourant ladite sortie (37) et la sortie (37) elle-même; et des moyens de support (33) pour supporter au moins lesdits moyens de stérilisation (41); ladite sortie (37) et lesdits moyens de support (33) pouvant être connectés respectivement à un circuit hydraulique et à un boîtier (32a) d'une unité (32) de distribution de produits alimentaires liquides; et étant **caractérisé en ce que** lesdits moyens de stérilisation (41) comprennent au moins une lampe ultraviolette (41) pour irradier ladite sortie (37) et ledit espace entourant ladite sortie.
